(19) Europäisches Patentamt
European Patent Office
Office européen des brevets

(11) Veröffentlichungsnummer: **0 177 054 B1**

(12) # EUROPÄISCHE PATENTSCHRIFT

(45) Veröffentlichungstag der Patentschrift: **08.02.89**

(51) Int. Cl.⁴: **C 07 D 213/70**, C 07 D 213/80, C 07 D 213/82, C 07 D 401/12, A 61 K 31/44, A 61 K 31/455

(21) Anmeldenummer: **85112597.1**

(22) Anmeldetag: **04.10.85**

(54) **Neue Pyridinderivate, Verfahren zu ihrer Herstellung und diese enthaltende pharmazeutische Präparate.**

(30) Priorität: **05.10.84 HU 377784**

(43) Veröffentlichungstag der Anmeldung: **09.04.86 Patentblatt 86/15**

(45) Bekanntmachung des Hinweises auf die Patenterteilung: **08.02.89 Patentblatt 89/6**

(84) Benannte Vertragsstaaten: **AT BE CH DE FR GB IT LI NL SE**

(56) Entgegenhaltungen:
**EP-A- 0 066 628**
**AT-B- 238 200**
**US-A- 4 260 620**

**CHEMICAL ABSTRACTS, Band 96, Nr. 5, 1 Februar 1982, Columbus, Ohio, USA. YOSHITOMI: "Pyridine derivatives", Seite 652, Spalte 2, Zusammenfassung Nr. 25096v**

(73) Patentinhaber: **RICHTER GEDEON VEGYESZETI GYAR R.T., Gyömröi ut 19-21, H-1475 Budapest X (HU)**

(72) Erfinder: **Ezer, Elemér, Lupény u. 6-8, H-1026 Budapest (HU)**
Erfinder: **Harsányi, Kálmán, Dr., Fodor u. 12, H-1126 Budapest (HU)**
Erfinder: **Vikár, Hajnalka, Süveg u. 16, H-1112 Budapest (HU)**
Erfinder: **Matuz, Judit, Ozgida u. 32, H-1025 Budapest (HU)**
Erfinder: **Szporny, László, Dr., Szabolcska u. 7, H-1114 Budapest (HU)**
Erfinder: **Cholnoky, Eszter, Dr., Bartók Béla u. 4, H-1111 Budapest (HU)**
Erfinder: **Kuthi, Csaba, Bürök u. 24, H-1124 Budapest (HU)**
Erfinder: **Trischler, Ferenc, Dr., Uttörö u. 16, H-1171 Budapest (HU)**
Erfinder: **Hegedüs, Béla, Dr., Bartók bBéla u. 82, H-1113 Budapest (HU)**
Erfinder: **Kápolnás, Márta, 525 tér 33, H-1173 Budapest (HU)**
Erfinder: **Kállay, Anna, Csertö u. 18-20, H-1144 Budapest (HU)**

(74) Vertreter: **Eitle, Werner, Dipl.-Ing. et al, Hoffmann, Eitle & Partner Patentanwälte Arabellastrasse 4, D-8000 München 81 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Pyridinderivate, Verfahren zu ihrer Herstellung und diese enthaltende pharmazeutische Präparate. Gegenstand der Erfindung sind Pyridinderivate der allgemeinen Formel IA

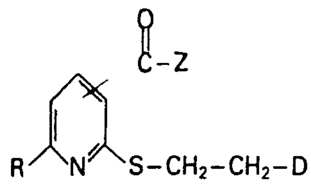

(IA)

worin

R ein Wasserstoffatom oder eine Alkylgruppe mit 1 bis 4 Kohlenstoffatomen bedeutet,

Z eine gegebenenfalls durch ein oder mehrere Halogenatom(e) und/oder Alkylgruppe(n) mit 1 bis 4 Kohlenstoffatomen substituierte Phenylgruppe darstellt,

D eine Hydroxylgruppe oder ein Halogenatom, oder eine –NH–E–Gruppe darstellt, worin E eine Gruppe der folgenden Formeln bedeutet:

(a) 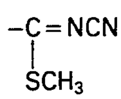

(b) 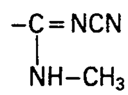 oder

(c) 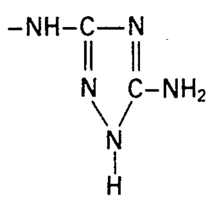

und der Substituent

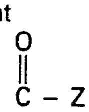

in der 3- oder 4-Position des Pyridinringes bindet, und ihre Säureadditionssalze.

Gemäss der Erfindung können die Verbindungen der allgemeinen Formel IA wie folgt hergestellt werden:

$a_1$) ein 2-Halogen-pyridin-Derivat der allgemeinen Formel II

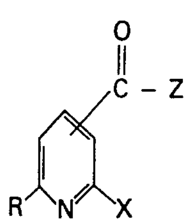

(II)

– wobei die Bedeutung von R und Z wie oben angegeben ist und X ein Halogenatom bedeutet – wird mit einer Thiolverbindung der allgemeinen Formel III

$$HS-CH_2-CH_2-D \qquad (III)$$

– wobei die Bedeutung von D die obige ist – oder mit deren Säureadditionssalz umgesetzt; oder

$a_2$) ein Pyridin-2-thion-Derivat der allgemeinen Formel IV

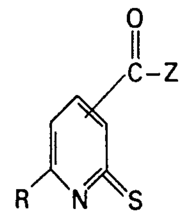

(IV)

– wobei die Bedeutung von Z und R wie oben ist – wird mit einem 2-Halogen-äthan-Derivat der allgemeinen Formel V

$$X-CH_2-CH_2-D \qquad (V)$$

– wobei die Bedeutung von D die obige und die von X Halogenatom ist – oder mit dessen Säureadditionssalz umgesetzt und gewünschtenfalls wird die erhaltene Verbindung der allgemeinen Formel IA zu einem Säureadditionssalz umgesetzt oder aus ihrem Säureadditionssalz freigesetzt.

Die Verbindungen der allgemeinen Formel IA sind teils therapeutisch aktiv, durch eine besonders wertvolle geschwürhemmende, schmerzlindernde, die Aggregation der Blutplättchen hemmende Wirkung gekennzeichnet, teils sind sie wertvolle Intermediäre bei der Herstellung von anderen therapeutisch aktiven Pyridinderivaten.

Die Erfindung betrifft ferner pharmazeutische Präparate, die die Verbindungen der allgemeinen Formel IA oder deren therapeutisch akzeptierbare Säureadditionssalze als Wirkstoffe enthalten.

In den obigen allgemeinen Formeln können in der Bedeutung von R und Z die Kohlenstoffketten der Alkylgruppen mit 1 – 4 Kohlenstoffatomen gerade oder verzweigt sein. Als Beispiel werden Methyl-, Äthyl-, n-Propyl-, i-Propyl-, n-Butyl-, sec-Butyl- oder tert-Butylgruppen genannt.

Z steht vorzugsweise für eine nicht substituierte oder mono- oder disubstituierte Phenylgruppe. Als Substituent sind besonders das Chloratom und die Methylgruppe vorteilhaft.

X kann als Halogenatom Fluor, Chlor-, Brom- oder Jodatom bedeuten, vorzugsweise Chlor- oder Bromatom.

Mit Verbindungen analoger Struktur befasst sich die unter der Nr. 56–100765 offengelegte japanische Patentanmeldung Nr. 55–2920, welche Pyridinderivate beschreibt, die überwiegend Alkyl- und Dialkyl-amino-alkoxy-Seitenketten in 2-Stellung enthalten. Der Anmeldung zufolge weisen diese Verbindungen eine Anti-Apomorphin-, Muskelrelaxans-, eine beruhigende und Hirnkreislauf beeinflussende Wirkung auf; die angegebenen Wirkungen werden jedoch nicht von pharmakologischen Daten untermauert. Weder die geschwürhemmende oder andere, früher beschriebene Wirkungen der Verbindungen noch ihre Verwendung in der pharmazeutischen Industrie als Intermediär werden erwähnt.

Pyridin-2-thiol-Derivate beschreibt die japanische Patentschrift Nr. 175 108. Einige Vertreter der sich von den erfindungsgemässen Verbindungen strukturell eindeutig unterscheidenden Pyridinderivate können zur Phagocytose von Leukocyten und Makrophagen und zur Behandlung von rheumatischen Entzündungen verwendet werden.

Nach der Verfahrensvariante $a_1$) der Erfindung lässt man die Verbindungen der allgemeinen Formeln II und III – wobei die Bedeutung der Substituenten die früher angegeben ist – in einem Lösungsmittel, vorzugsweise in Gegenwart von Säurebindemittel reagieren. Als Lösungsmittel können zweckmässigerweise Alkohole mit kurzen Kohlenstoffketten (1–4 Kohlenstoffatome), Wasser oder deren Gemische verwendet werden. Als Säurebindungsmittel werden vorzugsweise Alkalimetallhydroxyde, -karbonate, -alkoholate oder organische Basen, z. B. Triäthyl-amin oder quaternäre Ammoniumverbindungen verwendet. Die Reaktionstemperatur kann sich – abhängig von den Reagenzien, dem verwendeten Lösungsmittel und den eventuellen Nebenreaktionen – in breiten Grenzen bewegen, aber im Interesse des Erreichens der entsprechenden Reaktionsgeschwindigkeit ist es zweckmässig, bei einer Temperatur zwischen 25 °C und 80 °C zu arbeiten. Im Falle der Wahl eines entsprechenden Lösungsmittels können die anorganischen Salze am Ende der Reaktion durch Filtern entfernt werden. Nach Eindampfen des Reaktionsgemisches kann man die kristallinen Produkte durch Umkristallisieren reinigen, die als Base nicht kristallisierenden Stoffe hingegen sind durch Eindampfen der organischen Phase isolierbar, nachdem mit einem sich mit Wasser nicht vermischenden Lösungsmittel, z. B. mit chlorierten Kohlenwasserstoffen, Äthern oder Äthylacetat, extrahiert wurde, und gewünschtenfalls können sie durch Vakuumdestillation gereinigt werden. Aus den in Basenform nicht kristallisierenden Produkten können gut kristallisierende Säureadditionssalze, zweckmässigerweise Hydrochloride, hergestellt werden.

Das erfindungsgemässe Verfahren $a_1$) ist auch in saurem Milieu durchführbar. Dabei ist es zweckmässig, die Reagenzien in wässriger Salzsäurelösung bei einer dem Siedepunkt des Gemisches entsprechenden Temperatur reagieren zu lassen.

Im Falle des erfindungsgemässen Verfahrens $a_2$) lässt man die Verbindungen der allgemeinen Formeln IV und V im wesentlichen unter den der ersten Variante des Verfahrens $a_1$) entsprechenden Bedingungen, in Gegenwart einer Base reagieren.

Die im Verfahren $a_1$) als Ausgangsstoff verwendeten Verbindungen der allgemeinen Formel II sind teils bekannt (siehe die unter der Nr. 0032516 offengelegte europäische Patentanmeldung Nr. 80010027.2), teils sind sie mit bekannten chemischen Methoden leicht herstellbar (Org. Synth. Coll. Vol. 4, 88/1963/; Wolfenstein und Hartwich: Ber. 48, 2043 /1915/).

Die im Verfahren $a_1$) verwendeten Verbindungen der allgemeinen Formel III und im Verfahren $a_2$) verwendeten Verbindungen der allgemeinen Formel V sind bekannte, im Handel erhältliche Stoffe bzw. aus solchen auf bekannte Weise herstellbar.

Die Pyridin-2-thion-Derivate der allgemeinen Formel IV sind teils bekannt (Spanische Patentschriften Nr. 506366, 506367 und 506368), teils auf an sich bekannte Weise aus bekannten, im Handel erhältlichen Verbindungen herstellbar.

Der Stubstituent D der neuen Verbindungen der allgemeinen Formel IA kann auf bekannte Weise zu einem Substituenten umgebildet werden, der unter die früher gegebene Definition von D fällt.

Ist in der allgemeinen Formel IA die Bedeutung von D eine Hydroxylgruppe, so können die entsprechenden Halogenverbindungen auf bekannte Weise, durch Halogenieren, hergestellt werden. Für die Herstellung von Chlorverbindungen z. B. wird zweckmässigerweise Thionyl-chlorid verwendet.

Verbindungen der allgemeinen Formel IA, die als D eine Gruppe der allgemeinen Formel –NH–E und als E-Gruppe eine Gruppe der Formel (b) bzw. (c) enthalten, sind durch Reaktion der entsprechenden, als E-Gruppe eine Gruppe der Formel (a) enthaltenden Verbindung und Methylamin oder Hydrazinhydrat herstellbar. Es ist zweckmässig, die Reaktion mit Methylamin in wässrigem Milieu durchzuführen, während bei der Anwendung des Hydrazinhydrats als Reagens dessen Überschuss gleichzeitig als Lösungsmittel dienen kann, aber es können auch andere, protische Lösungsmittel benutzt werden.

Die Verbindungen der allgemeinen Formel IA sind wertvolle Intermediäre, die z. B. bei der Herstellung der in der parallel eingereichten europäischen Patentanmeldung EP-A-0177907 erwähnten, in der Geschwürtherapie verwendbaren, durch gastrocytoprotektive Wirkung ausgezeichneten neuen Verbindungen Verwendung finden. So sind durch die Reaktion von erfindungsgemässen Verbindungen, die als D ein Halogenatom besitzen, und von entsprechenden Aminen leicht die entsprechenden Verbindungen, die an der Stelle 3 oder 4 des Pyridinringes eine Gruppe der allgemeinen Formel

$$-\underset{\underset{O}{\|}}{C}-Z$$

(Z besitzt die frühere Bedeutung) enthalten, herstellbar.

Die durch das erfindungsgemässe Verfahren hergestellten Verbindungen der allgemeinen Formel IA können gewünschtenfalls zu einem Säureadditionssalz umgesetzt werden. Die Säureadditionssalzbildung kann in einem neutralen Lösungsmittel durchgeführt werden, z. B. in einem

aliphatischen Alkohol mit 1–6 Kohlenstoffatomen, so dass die Verbindung der allgemeinen Formel IA im obigen Lösungsmittel gelöst wird, dann wird zur Lösung so lange die entsprechende Säure bzw. deren mit dem obigen Lösungsmittel bereitete Lösung gegeben, bis der pH-Wert des Gemisches auf sauer umschlägt. Danach isoliert man das ausgeschiedene Säureadditionssalz aus dem Reaktionsgemisch auf eine geeignete Weise, beispielsweise durch Filtern.

Untersuchung von durch saures Äthanol ausgelösten Magenschäden (cytoprotektive Wirkung):

Für die Untersuchungen wurden weibliche RG-Wistar-Ratten mit einem Gewicht von 120–150 g verwendet. Die Tiere liess man 24 Stunden lang hungern, während sie Wasser bekamen. Für die Irritierung des Magens wurde ein Gemisch von 1 ml konzentrierter Salzsäure und 50 ml absolutem Äthanol in einer Dosis von 0,5 ml/g Körpergewicht oral verwendet. Die zu untersuchenden Verbindungen wurden ebenfalls oral, 30 Minuten vor der Säure-Äthanol-Behandlung verabreicht, die Tiere wurden eine Stunde später durch Äther eingeschläfert. Der Magen wurde entfernt, dann geöffnet. Nach Reinigen wurde das Nassgewicht des Magens gemessen, danach wurde – im Interesse der Bestimmung des Magenödems – der Unterschied zwischen dem erhaltenen Nassgewicht und dem Nassgewicht des Magens der unbehandelten (Kontroll-) Tiere ausgerechnet. Danach wurden die Magen getrocknet und die Magenschäden visuell betrachtet. Zur Charakterisierung des Ausmasses der Schäden wurde die in mm angegebene durchschnittliche Länge der Magenschädigung verwendet. Die statistische Auswertung der Ergebnisse erfolgte mit der Student-Probe.

Man erhielt die folgenden Ergebnisse:

| Untersuchte Verbindung | Magenödem-Hemmung $ED_{50}$ [mg/kg p.o.] | Hämorrhagische Schädigung –Hemmung $ED_{50}$ [mg/kg p.o.] |
|---|---|---|
| A | 40,0 | 50,0 |

Verbindung A ist das N-[2-(/3-Benzoyl-2-pyridyl/-thio)-äthyl]-N'-cyano-S-methyl-isothiokarbamid.

Der Wirkstoff der allgemeinen Formel IA kann mit in der Pharmazie gebräuchlichen, zur parenteralen oder enteralen Dosierung geeigneten, nicht toxischen, inerten festen oder flüssigen Trägerstoffen und/oder Hilfsstoffen zu pharmazeutischen Präparaten konvertiert werden. Als Trägerstoff kann man z.B. Wasser, Gelatine, Laktose, Milchzucker, Stärke, Pektin, Magnesiumstearat, Stearinsäure, Talcum, Pflanzenöle (z.B. Erdnussöl, Olivenöl) usw. verwenden. Das pharmazeutische Präparat kann in gebräuchlicher Form, so besonders in fester Form, z.B. in Form von runden oder eckigen Tabletten, Dragees, Kapseln (z.B. Gelatinekapseln), Pilen, Zäpfchen usw. angefertigt sein. die Menge des festen Trägerstoffes kann sich innerhalb breiter Grenzen bewegen, vorteilhaft ist ein Wert zwischen etwa 25 mg und 1 g. Die Präparate können gegebenenfalls die üblichen pharmazeutischen Hilfsstoffe, z.B. Konservierungsstoffe, Stabilisierungsstoffe, Netzstoffe, Emulgierstoffe usw. enthalten. Sie können mit üblichen Methoden, z.B. im Fall der festen Präparate durch Sieben der Komponenten, Mischen, Granulieren und Pressen der Komponenten, hergestellt werden. Die Präparate können weiteren pharmakotechnischen Operationen, z.B. Sterilisation, unterworfen werden.

Die durch die allgemeine Formel IA charakterisierten Verbindungen sind nach der Nomenklatur der Chemical Abstracts substituierte Methanone, Methanole oder Säureamide, ihrer vom Aspekt der Bezeichnung aus betonten funktionellen Gruppe entsprechend. Der Einfachheit und Überschaubarkeit halber werden die Verbindungen in jedem der folgenden Beispiele – aufgrund des in jedem von ihnen vorkommenden Pyridinringes – als substituierte Pyridine erwähnt, jedoch wird auch die Bezeichnung nach der Nomenklatur der Chemical Abstracts angegeben.

Die Erfindung wird durch die folgenden Beispiele veranschaulicht, ohne dabei ihren Schutzumfang auf diese Beispiele einzuschränken.

Beispiel 1

N-[2-(/3-Benzoyl-2-pyridyl/-thio)-äthyl]-N'-oyano-S-methyl-isothiokarbamid

4,42 g (0,015 Mol) 2-[(2-Amino-äthyl)-thio]-3-benzoyl-pyridin·HCl werden in 10 $cm^3$ Wasser suspendiert, mit Natriumkarbonat zu einer Base umgebildet, dann gibt man die mit 7,5 $cm^3$ Äthanol bereitete Lösung von 2,35 g (0,015 Mol; 93,5%ig) Cyanimino-dithiokohlensäure-dimethylester dazu und kocht das Reaktionsgemisch eine Stunde lang. Nach dem Abkühlen wird der ausgeschiedene kristalline Stoff gefiltert, dann aus Acetonitril umkristallisiert. Man erhält 4,05 g (75,7%) N-[2-(/3-Benzoyl-2-pyridyl/-thio)-äthyl]-N'-cyano-S-methyl-isothiokarbamid, dessen Schmelzpunkt bei 180–181 °C liegt.

Analysenergebnisse, auf der Summenformel $C_{17}H_{16}N_4OS_2$ (Mg.: 356,46) basierend:

errechnet: C% = 57,28, H% = 4,52, N% = 15,72, S% = 17,99;

gefunden: C% = 57,32, H% = 4,32, N% = 15,87, S% = 17,55.

IR-Spektrum (KBr): 3270 $cm^{-1}$ –NH, 1633 $cm^{-1}$ >C=O, 1540 $cm^{-1}$ >C=N–, 1593, 782, 753, 708 $cm^{-1}$ –Ar

NMR-Spektrum (DMSO $d_6$): 2,5 ppm s $-S-CH_3$, 3,4 ppm m $-S-CH_2-$ und $-N-CH_2-$, 7,2 ppm 2×d Pyridin 5–H, 7,5 ppm m Phenylring, 7,7 ppm 2×d Pyridin 4–H, 8,4 ppm $^x$b –NH, 8,6 ppm 2×d Pyridin 6–H

Beispiel 2

N'-[2-(/3-Benzoyl-2-pyridyl/-thio)-äthyl]-N-cyano-N"-methyl-guanidin

6,63 g (0,051 Mol) N-Cyano-N,S-dimethyl-isothiokarbamid werden in 100 ml Äthanol kochend aufgelöst, es wird die mit 20 $cm^3$ Wasser bereitete Lösung von 5,83 g (0,051 Mol) Cysteamin·HCl tropfenweise dazugegeben, dann werden die mit 16 $cm^3$ Wasser bereitete Lösung von 4,11 g (0,103 Mol) Natriumhydroxyd und das Reaktionsgemisch eine Stunde lang gekocht. Danach gibt man die mit 10 $cm^3$ Äthanol bereitete Lösung von 11,18 g (0,051 Mol) 3-Benzoyl-2-chlor-pyridin dazu, und es wird 30 Stunden lang gekocht, dann eingedampft. Der Rest wird in Dichlormethan gelöst und mit Wasser extrahiert. Die Dichlormethan-Phase wird eingedampft, der Rest mit Isopropanol verrieben, gefiltert, getrocknet und aus Isopropanol umkristallisiert. Man erhält 9,3 g (53,3%) N'-[2-(/3-Benzoyl-2-pyridyl/-thio)-äthyl]-N-cyano-N''-methyl-guanidin, dessen Schmelzpunkt bei 132 – 133 °C liegt.

Analysenergebnisse, auf der Summenformel $C_{17}H_{17}N_5OS$ (Mg.: 339,42) basierend:

errechnet: C% = 60,16, H% = 5,05, N% = 20,63, S% = 9,45;

gefunden: C% = 60,17, H% = 4,92, N% = 20,76, S% = 9,37.

IR-Spektrum (KBr): 3270 $cm^{-1}$ >NH, 2170 $cm^{-1}$ >C=N, 1654 $cm^{-1}$ >C=O, 1595 $cm^{-1}$ >C=N–, 788, 750, 708 $cm^{-1}$ –Ar

NMR-Spektrum ($CDCl_3$): 2,8 ppm d→ s >N–$CH_3$, 3,3 ppm m –S–$CH_2$– und –N–$CH_2$–, 6,6 ppm $^x$m NH, 7,1 ppm q Pyridin 5–H, 7,5 ppm m Phenylring und Pyridin 4–H, 8,5 ppm 2×d Pyridin 6–H

Beispiel 3

Phenyl-2-{[2-/(5-imino-1,2-dihydro-1,2,4-triazol-3-yl)-amino/-äthyl]-thio}-pyrid-3-yl-keton 2-{[2-/(5-Imino-1,2-dihydro-1,2,4-triazol-3-yl)-amino/-äthyl]-thio}-3-pyridinyl-phenyl-methanon

5,5 g (0,015 Mol) N-[2-(/3-Benzoyl-2-pyridyl/-thio)-äthyl]-N'-cyano-S-methyl-isothiokarbamid werden eine Stunde lang mit 16 $cm^3$ (16,48 g; 0,33 Mol) Hydrazinhydrat gekocht, dann wir das Reaktionsgemisch im Vakuum eingedampft. Der Rest wird aus wässrigem Dimethyl-formamid umkristallisiert. Man erhält 2,8 g (54,8%) Phenyl-2-{[2-/(5-imino-1,2-dihydro-1,2,4-triazol-3-yl)-amino/-äthyl]-thio}-pyrid-3-yl-keton, dessen Schmelzpunkt 230 – 232 °C beträgt.

Analysenergebnisse, auf der Summenformel $C_{16}H_{16}N_6OS$ (Mg.: 340,40) basierend:

errechnet: C% = 56,45, H% = 4,74, N% = 24,69, O% = 4,70, S% = 9,62;

gefunden: C% = 56,38, H% = 4,59, N% = 24,65, O% = 4,82, S% = 9,84.

IR-Spektrum (KBr): 3420, 3320, 3230, 3110 $cm^{-1}$ >NH, 1640 $cm^{-1}$ >C=O, 1570 $cm^{-1}$ >C=N–, 1602, 804, 751, 692 $cm^{-1}$ –Ar

NMR-Spektrum ($CDCl_3$): 3,3 ppm b –S–$CH_2$– und –N–$CH_2$–, 5,0–5,5 ppm $^x$b >NH, 6,1 ppm $^x$s –$NH_2$, 6,9–7,3 ppm m Phenylring und Pyridin 4,5–H, 8,3 ppm 2×d Pyridin 6–H

Beispiel 4

3-Benzoyl-2-[(2-hydroxy-äthyl)-thio]-pyridin·HCl [2-(/2-Hydroxy-äthyl/-thio)-3-pyridinyl]phenyl-methanon·HCl

10,88 g (0,05 Mol) 3-Benzoyl-2-chlor-pyridin und 3,84 g (0,055 Mol) 2-Merkapto-äthanol werden in 30 $cm^3$ Äthanol gelöst, dann wird die mit 30 $cm^3$ Äthanol bereitete Lösung von 2,2 g (0,055 Mol) Natriumhydroxyd dazugegeben, und das Reaktionsgemisch wird 2,5 Stunden lang gekocht. Dann werden erneut 0,78 g (0,01 Mol) Merkaptoäthanol dazugegeben und weitere 1,5 Stunden lang gekocht. Das anorganische Salz wird ausgefiltert, die Lösung eingedampft, der Rest in Wasser gelöst und mit 1,2-Dichloräthan extrahiert.

Die organische Phase wird mit 2 N Natriumhydroxyd, dann mit Wasser gewaschen, eingedampft, und mit salzsaurem Äthylacetat wird daraus ein Salz gebildet. Der Schmelzpunkt des erhaltenen 2-[(Hydroxyäthyl)-thio]-3-benzoyl-pyridin·HCl beträgt 126–127 °C.

Analysenergebnisse, auf der Summenformel $C_{14}H_{13}NO_2S.HCl$ (Mg.: 295,78) basierend:

errechnet: C% = 56,85, N% = 4,74, S% = 10,84, Cl% = 11,99;

gefunden: C% = 56,58, N% = 4,68, S% = 10,53, Cl% = 11,63.

IR-Spektrum (KBr): 3360 $cm^{-1}$ –OH, 3100 – 2100 $cm^{-1}$ –$N^+H$, 1660 $cm^{-1}$ C=O, 1600, 800, 758, 710 $cm^{-1}$ –Ar

NMR-Spektrum ($CDCl_3$): 3,6 ppm t –S–$CH_2$–, 3,9 ppm t –O–a-$CH_2$–, 7,4 – 7,8 ppm m Phenylring und Pyridin 5–H, 8,0 ppm 2×d Pyridin 4–H, 8,8 ppm 2×d Pyridin 6–H, 9,5 ppm $^x$s –OH und –$NH^+$

**Patentansprüche für die Vertragsstaaten: BE, CH, DE, FR, GB, IT, LI, NL, SE**

1. Pyridinderivate der allgemeinen Formel IA

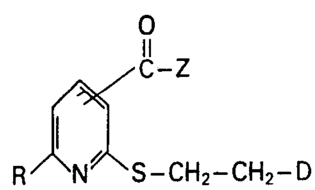

(IA)

worin

R ein Wasserstoffatom oder eine Alkylgruppe mit 1 bis 4 Kohlenstoffatomen bedeutet,

Z eine gegenbenfalls durch ein oder mehrere Halogenatom(e) und/oder Alyklgruppe(n) mit 1 bis 4 Kohlenstoffatomen substituierte Phenylgruppe darstellt,

D eine Hydroxylgruppe oder ein Halogenatom, oder eine –NH–E–Gruppe darstellt, worin E eine Gruppe der folgenden Formeln bedeutet:

(a) 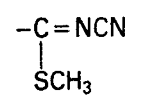

(b) 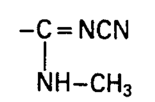 oder

(c) 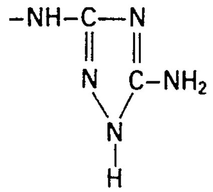

und der Substituent

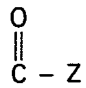

in der 3- oder 4-Position des Pyridinringes bindet, und ihre Säureadditionssalze.

2. Pharmazeutisches Präparat, dadurch gekennzeichnet, dass es als Wirkstoff mindestens eine Verbindung der Formel IA oder ihr therapeutisch annehmbares Säureadditionssalz zusammen mit üblichen Trägerstoffen umfasst.

3. Verfahren zur Herstellung von Pyridinderivaten der allgemeinen Formel IA

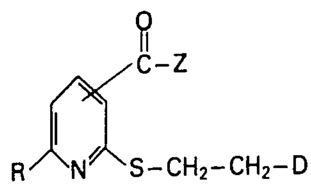

(IA)

worin

R ein Wasserstoffatom oder eine Alkylgruppe mit 1 bis 4 Kohlenstoffatomen bedeutet,

Z eine gegebenenfalls durch ein oder mehrere Halogenatom(e) und/oder Alkylgruppe(n) mit 1 bis 4 Kohlenstoffatomen substituierte Phenylgruppe darstellt,

D eine Hydroxylgruppe oder ein Halogenatom, oder eine –NH–E-Gruppe darstellt, worin E eine Gruppe der folgenden Formeln bedeutet:

(a) 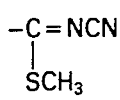

(b) 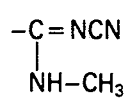 oder

(c) 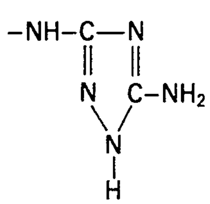

und der Substituent

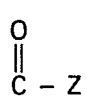

die 3- oder 4-Position des Pyridinringes bindet, und ihrer Säureadditionssalze, dadurch gekennzeichnet, dass entweder a1) eine Verbindung der Formel II

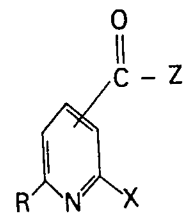

(II)

wobei R und Z die vorstehend angegebenen Bedeutungen haben, und X ein Halogenatom darstellt, mit einer Thiolverbindung der allgemeinen Formel III

$$HS-CH_2-CH_2-D \quad (III)$$

worin D die vorstehend angegebene Bedeutung hat, oder mit deren Säureadditionssalz umgesetzt wird; oder a2) ein Pyridin-2-thion-Derivat der allgemeinen Formel IV

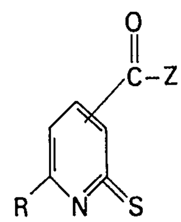

(IV)

worin Z und R die vorstehend angegebenen Bedeutungen haben, mit einem 2-Halogen-ethan-Derivat der allgemeinen Formel V

$$X-CH_2-CH_2-D \quad (V)$$

worin D die vorstehend angegebene Bedeutung hat, und X ein Halogenatom bedeutet, oder dessen Säureadditionssalz umgesetzt wird, und gewünschtenfalls die erhaltene Verbindung der allgemeinen Formel IA zu einem Säureadditionssalz umgewandelt oder aus ihrem Säureadditionssalz freigesetzt wird.

4. Verfahren nach Anspruch 3, Variante a1), dadurch gekennzeichnet, dass die Reaktion in einem Lösungsmittel in Gegenwart eines Säurebindemittels durchgeführt wird.

5. Verfahren nach Anspruch 3, Variante a2), dadurch gekennzeichnet, dass die Reaktion in konzentrierter Salzsäurelösung bei einer Temperatur, die dem Siedepunkt entspricht, durchgeführt wird.

6. Verfahren nach Anspruch 3, Variante a2), dadurch gekennzeichnet, dass die Reaktion in einem Alkohol mit kurzer Kohlenstoffkette in Gegenwart von Alkalimetallhydroxid stattfindet.

7. Verfahren nach Anspruch 3 zur Herstellung von Verbindungen der allgemeinen Formel IA, worin D eine Gruppe der allgemeinen Formel –NH–E und E eine Gruppe der Formel (b) ist, R und Z die vorstehend angegebenen Bedeutungen haben, dadurch gekennzeichnet, dass eine Verbindung der allgemeinen Formel IA, in der D eine –NH–E-Gruppe bedeutet, und darin E eine Gruppe

der Formel (a) ist, mit Methylamin umgesetzt wird.

8. Verfahren nach Anspruch 3 zur Herstellung von Verbindungen der allgemeinen Formel IA, worin D eine Gruppe der allgemeinen Formel –NH–E und E eine Gruppe der Formel (c) ist, R und Z die in Anspruch 1 angegebenen Bedeutungen haben, dadurch gekennzeichnet, dass eine Verbindung der allgemeinen Formel IA, worin D eine –NH–E-Gruppe und darin E eine Gruppe der Formel (a) ist, mit Hydrazinhydrat umgesetzt wird.

**Patentansprüche für den Vertragsstaat: Österreich**

1. Verfahren zur Herstellung von Pyridinderivaten, gekennzeichnet durch die Synthese einer Verbindung der allgemeinen Formel IA

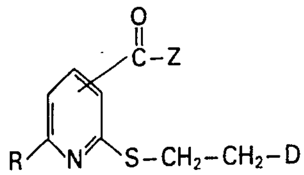

(IA)

worin

R ein Wasserstoffatom oder eine Alkylgruppe mit 1 bis 4 Kohlenstoffatomen bedeutet,

Z eine gegenbenfalls durch ein oder mehrere Halogenatom(e) und/oder Alyklgruppe(n) mit 1 bis 4 Kohlenstoffatomen substituierte Phenylgruppe darstellt,

D eine Hydroxylgruppe oder ein Halogenatom, oder eine –NH–E–Gruppe darstellt, worin E eine Gruppe der folgenden Formeln bedeutet:

(a) 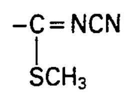

(b) 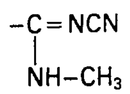 oder

(c) 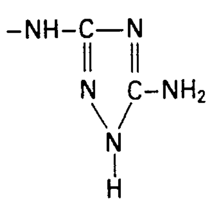

und der Substituent

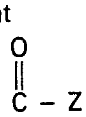

in der 3- oder 4-Position des Pyridinringes bindet, und ihre Säureadditionssalze.

2. Verfahren zur Herstellung von Pyridinderivaten der allgemeinen Formel IA

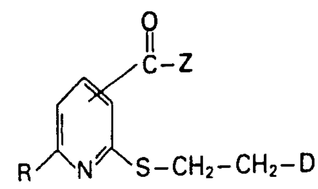

(IA)

worin

R ein Wasserstoffatom oder eine Alkylgruppe mit 1 bis 4 Kohlenstoffatomen bedeutet,

Z eine gegebenenfalls durch ein oder mehrere Halogenatom(e) und/oder Alkylgruppe(n) mit 1 bis 4 Kohlenstoffatomen substituierte Phenylgruppe darstellt,

D eine Hydroxylgruppe oder ein Halogenatom, oder eine –NH–E-Gruppe darstellt, worin E eine Gruppe der folgenden Formeln bedeutet:

(a) 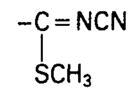

(b) 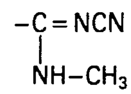 oder

(c) 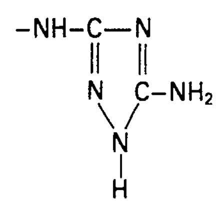

und der Substituent

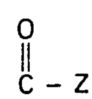

die 3- oder 4-Position des Pyridinringes bindet, und ihrer Säureadditionssalze, dadurch gekennzeichnet, dass entweder a1) eine Verbindung der Formel II

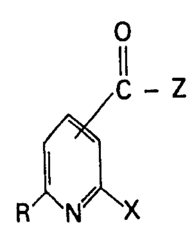

(II)

wobei R und Z die vorstehend angegebenen Bedeutungen haben, und X ein Halogenatom darstellt, mit einer Thiolverbindung der allgemeinen Formel III

$$HS{-}CH_2{-}CH_2{-}D \qquad (III)$$

worin D die vorstehend angegebene Bedeutung hat, oder mit deren Säureadditionssalz umgesetzt wird; oder a2) ein Pyridin-2-thion-Derivat der allgemeinen Formel IV

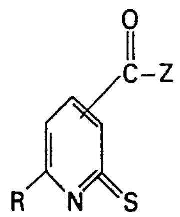

(IV)

worin Z und R die vorstehend angegebenen Bedeutungen haben, mit einem 2-Halogen-ethan-Derivat der allgemeinen Formel V

$$X-CH_2-CH_2-D \qquad (V)$$

worin D die vorstehend angegebene Bedeutung hat, und X ein Halogenatom bedeutet, oder dessen Säureadditionssalz umgesetzt wird, und gewünschtenfalls die erhaltene Verbindung der allgemeinen Formel IA zu einem Säureadditionssalz umgewandelt oder aus ihrem Säureadditionssalz freigesetzt wird.

3. Verfahren nach Anspruch 1 und 2 Variante a1), dadurch gekennzeichnet, dass die Reaktion in einem Lösungsmittel in Gegenwart eines Säurebindemittels durchgeführt wird.

4. Verfahren nach Anspruch 1 und 2, Variante a1), dadurch gekennzeichnet, dass die Reaktion in konzentrierter Salzsäurelösung bei einer Temperatur, die dem Siedepunkt des Gemisches entspricht, durchgeführt wird.

5. Verfahren nach Anspruch 1 und 2, Variante a2), dadurch gekennzeichnet, dass die Reaktion in einem Alkohol mit kurzer Kohlenstoffkette in Gegenwart von Alkalimetallhydroxid stattfindet.

6. Verfahren nach Anspruch 1 und 2 zur Herstellung von Verbindungen der allgemeinen Formel IA, worin D eine Gruppe der allgemeinen Formel –NH–E und E eine Gruppe der Formel (b) ist, R und Z die vorstehend angegebenen Bedeutungen haben, dadurch gekennzeichnet, dass eine Verbindung der allgemeinen Formel IA, in der D eine –NH–E-Grupe bedeutet, und darin E eine Gruppe der Formel (a) ist, mit Methylamin umgesetzt wird.

7. Verfahren nach Anspruch 1 und 2 zur Herstellung von Verbindungen der allgemeinen Formel IA, worin D eine Gruppe der allgemeinen Formel –NH–E und E eine Gruppe der Formel (c) ist, R und Z die in Anspruch 1 angegebenen Bedeutungen haben, dadurch gekennzeichnet, dass eine Verbindung der allgemeinen Formel IA, worin D eine –NH–E-Gruppe und darin E eine Gruppe der Formel (a) ist, mit Hydrazinhydrat umgesetzt wird.

**Claims for the Contracting States: BE, CH, DE, FR, GB, IT, LI, NL, SE**

1. Pyridine derivatives of the general formula IA

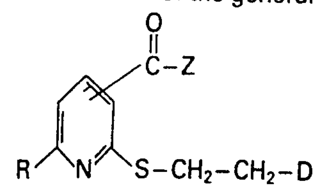

(IA)

wherein

R denotes a hydrogen atom or an alkyl group having 1 to 4 carbon atoms,

Z represents a phenyl group which is optionally substituted by one or more halogen atom(s) and/or alkyl group(s) having 1 to 4 carbon atoms,

D represents a hydroxyl group or a halogen atom, or an –NH–E group, wherein E denotes a group of the following formulae:

(a) 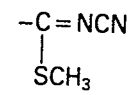

(b) 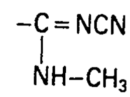 or

(c) 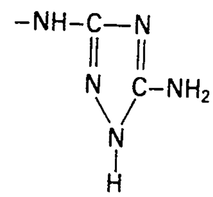

and the substituent

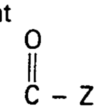

bonds in the 3- or 4-position of the pyridine ring, and their acid addition salts.

2. Pharmaceutical preparation characterized in that it contains as an active compound at least one compound of the formula IA or its therapeutically acceptable acid addition salt together with customary excipients.

3. Process for the preparation of pyridine derivatives of the general formula IA

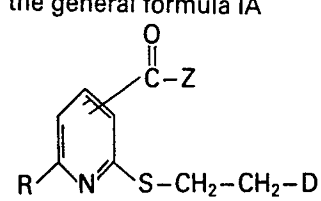

(IA)

wherein

R denotes a hydrogen atom or an alkyl group having 1 to 4 carbon atoms,

Z represents a phenyl group which is optionally substituted by one or more halogen atom(s) and/or alkyl group(s) having 1 to 4 carbon atoms,

D represents a hydroxyl group or a halogen atom, or an –NH–E group, wherein E denotes a group of the following formulae:

(a) 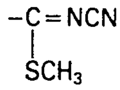

(b) 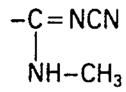 or

(c) 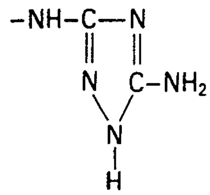

and the substituent

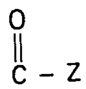

bonds in the 3- or 4-position of the pyridine ring, and their acid addition salts, characterized in that either a1) a compound of the formula II

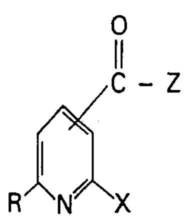

(II)

where R and Z have the meanings previously indicated and X represents a halogen atom, is reacted with a thiol compound of the general formula III

$HS-CH_2-CH_2-D$ (III)

wherein D has the meaning previously indicated, or with its acid addition salt; or a2) a pyridine-2-thione derivative of the general formula IV

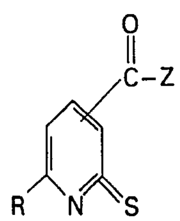

(IV)

wherein Z and R have the meanings previously indicated, is reacted with a 2-haloethane derivative of the general formula V

$X-CH_2-CH_2-D$ (V)

wherein D has the meaning previously indicated, and X denotes a halogen atom, or its acid addition salt, and if desired, the compound of the general formula IA obtained is converted to an acid addition salt or is released from its acid addition salt.

4. Process according to claim 3, variant a1), characterized in that the reaction is carried out in a solvent in the presence of an acid-binding agent.

5. Process according to claim 3, variant a1), characterized in that the reaction is carried out in concentrated hydrochloric acid solution at a temperature which corresponds to the boiling point of the mixture.

6. Process according to claim 3, variant a2), characterized in that the reaction takes place in an alcohol having a short carbon chain in the presence of alkali metal hydroxide.

7. Process according to claim 3 for the preparation of compounds of the general formula IA, wherein D is a group of the general formula –NH–E and E is a group of the formula (b), and R and Z have the meanings previously indicated, characterized in that a compound of the general formula IA, in which D denotes an –NH–E-group, and in which E is a group of the formula (a), is reacted with methylamine.

8. Process according to claim 3 for the preparation of compounds of the general formula IA, wherein D is a group of the general formula –NH–E and E is a group of the formula (c), and R and Z have the meanings indicated in claim 1, characterized in that a compound of the general formula IA, wherein D is an –NH–E-group and in which E is a group of the formula (a), is reacted with hydrazine hydrate.

**Claims for the Contracting State: AT**

1. Process for the preparation of pyridine derivatives, characterized by the synthesis of a compound of the general formula IA

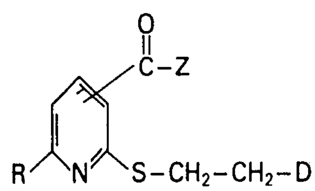

(IA)

where

R denotes a hydrogen atom or an alkyl group having 1 to 4 carbon atoms,

Z represents a phenyl group which is optionally substituted by one or more halogen atom(s) and/or alkyl group(s) having 1 to 4 carbon atoms,

D represents a hydroxyl group or a halogen atom, or an –NH–E group, wherein E denotes a group of the following formulae:

(a) 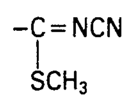

(b) 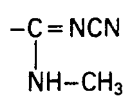 or

(c) 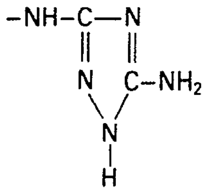

and the substituent

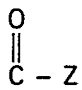

bonds in the 3- or 4-position of the pyridine ring, and their acid addition salts.

2. Process for the preparation of pyridine derivatives of the general formula IA

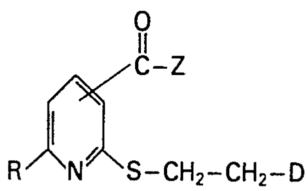

(IA)

where

R denotes a hydrogen atom or an alkyl group having 1 to 4 carbon atoms,

Z represents a phenyl group which is optionally substituted by one or more halogen atom(s) and/or alkyl group(s) having 1 to 4 carbon atoms,

D represents a hydroxyl group or a halogen atom, or an –NH–E group, wherein E denotes a group of the following formulae:

(a) 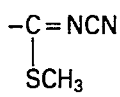

(b) 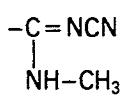 or

(c) 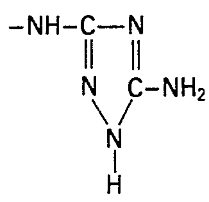

and the substituent

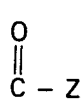

bonds in the 3- or 4-position of the pyridine ring, and their acid addition salts, characterized in that either a1) a compound of the formula II

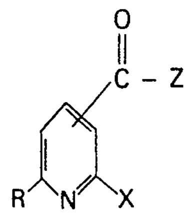

(II)

where R and Z have the meanings previously indicated and X represents a halogen atom, is reacted with a thiol compoud of the general formula III

$$HS-CH_2-CH_2-D \quad \text{(III)}$$

wherein D has the meaning previously indicated, or with its acid addition salt; or a2) a pyridine-2-thione derivative of the general formula IV

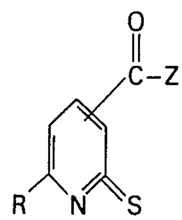

(IV)

wherein Z and R have the meanings previously indicated, is reacted with a 2-haloethane derivative of the general formula V

$$X-CH_2-CH_2-D \quad \text{(V)}$$

wherein D has the meaning previously indicated, and X denotes a halogen atom, or its acid addition salt, and, if desired, the compound of the general formula (IA) obtained is converted to an acid addition salt or is released from its acid addition salt.

3. Process according to claim 1 and 2, variant a1), chatacterized in that the reaction is carried out in a solvent in the presence of an acid-binding agent.

4. Process according to claim 1 and 2, variant a1), characterized in that the reaction is carried out in concentrated hydrochloric acid solution at a temperature which corresponds to the boiling point of the mixture.

5. Process according to claim 1 and 2, variant a2), characterized in that the reaction takes place in an alcohol having a short carbon chain in the presence of alkali metal hydroxide.

6. Process according to claim 1 and 2 for the preparation of compounds of the general formula IA, wherein D is a group of the general formula –NH–E and E is a group of the formula (b), and R and Z have the meanings previously indicated, characterized in that a compound of the general formula IA, in which D denotes an –NH–E-group, and in which E is a group of the formula (a), is reacted with methylamine.

7. Process according to claim 1 and 2 for the preparation of compounds of the general formula IA, wherein D is a group of the general formula

–NH–E and E is a group of the fromula (c), and R and Z have the meanings indicated in claim 1, characterized in that a compound of the general formula IA, wherein D is an –NH–E-group and in which E is a group of the formula (a), is reacted with hydrazine hydrate.

**Revendications pour les Etats contractants: BE, CH, DE, FR, GB, IT, LI, NL, SE**

1. Dérivés de la pyridine répondant à la formule générale IA

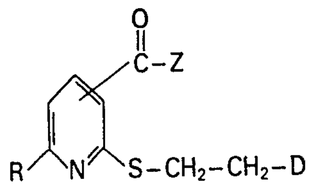

(IA)

dans laquelle

R représente un atome d'hydrogène ou un groupe alkyle en C1–C4,

Z représente un groupe phényle éventuellement substitué par un ou plusieurs atomes d'halogènes et/ou groupes alkyle en C1–C4,

D représente un groupe hydroxy ou un atome d'alogène ou un groupe –NH–E dans lequel E représente un groupe répondant aux formules suivantes:

(a) 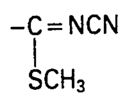

(b) 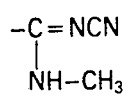 ou

(c) 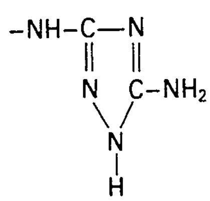

et le substituant

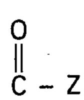

est fixé en position 3 ou 4 du cycle de pyridine, et leurs sels formés par addition avec des acides.

2. Composition pharmaceutique caractérisée en ce qu'elle contient en tant que substance active au moins un composé de formule IA ou un sel d'un tel composé formé par addition avec un acide acceptable pour l'usage pharmaceutique, avec des véhicules usuels.

3. Procédé de préparation des dérivés de la pyridine répondant à la formule générale IA

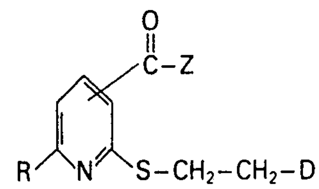

(IA)

dans laquelle

R représente un atome d'hydrogène ou un groupe alkyle en C1–C4,

Z représente un groupe phényle éventuellement substitué par un ou plusieurs atomes d'halogènes et/ou groupes alkyle en C1–C4,

D représente un groupe hydroxy ou un atome d'halogène, ou un groupe –NH–E dans lequel E représente un groupe répondant aux formules suivantes:

(a) 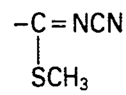

(b) 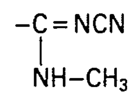 ou

(c) 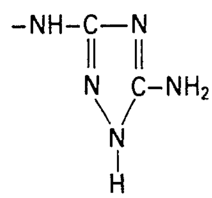

et le substituant

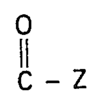

est fixé en position 3 ou 4 du cycle de pyridine, et de leurs sels formés par addition avec des acides, caractérisé en ce que: a1) on fait réagir un composé de formule II

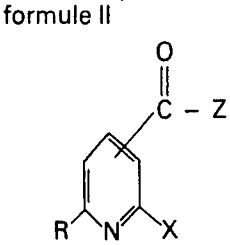

(II)

dans laquelle R et Z ont les significations indiquées ci-dessus, et X représente un atome d'halogène, avec un thiol de formule générale III

$$HS-CH_2-CH_2-D \qquad (III)$$

dans laquelle D a les significations indiquées ci-dessus, ou avec un sel d'un tel composé formé par addition avec un acide; ou bien a2) on fait réagir une pyridine-2-thione de formule générale IV

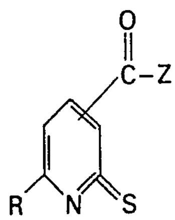

(IV)

dans laquelle R et Z ont les significations indiquées ci-dessus, avec un dérivé de 2-halogéno-éthane de formule générale V

$$X-CH_2-CH_2-D \qquad (V)$$

dans laquelle D a les significations indiquées ci-dessus et X représente un atome d'halogène, ou un sel de ce composé formé par addition avec un acide, et si on le désire, on convertit un composé obtenu répondant à la formule générale IA en un sel formé par addition avec un acide ou un libère un tel composé à partir d'un sel d'addition avec un acide.

4. Procédé selon la revendication 3, variante a1), caractérisé en ce que la réaction est effectuée dans un solvant en présence d'un accepteur d'acide.

5. Procédé selon la revendication 3, variante a1), caractérisé en ce que la réaction est effectuée dans une solution concentrée d'acide chlorhydrique à une température correspondant au point d'ébullition du mélange.

6. Procédé selon la revendication 3, variante a2), caractérisé en ce que la réaction est effectuée dans un alcool inférieur en présence d'un hydroxyde de métal alcalin.

7. Procédé selon la revendication 3, pour la préparation des composés de formule générale IA dans laquelle D représente un groupe de formule générale –NH–E et E représente un groupe de formule (b), R et Z ont les significations indiquées ci-dessus, caractérisé en ce que l'on fait réagir un composé de formule générale IA dans laquelle D représente un groupe –NH–E dans lequel E représente un groupe de formule (a), avec la méthylamine.

8. Procédé selon la revendication 3, pour la préparation des composés de formule générale IA dans laquelle D représente un groupe de formule générale –NH–E et E représente un groupe de formule (c), R et Z ayant les significations indiquées ci-dessus, caractérisé en ce que l'on fait réagir un composé de formule générale IA dans laquelle D représente un groupe –NH–E dans lequel E représente un groupe de formule (a), avec l'hydrate d'hydrazine.

**Revendications pour l'Etat contractant: AT**

1. Procédé de préparation de dérivés de la pyridine, caractérisé en ce que l'on prépare un composé de formule générale IA

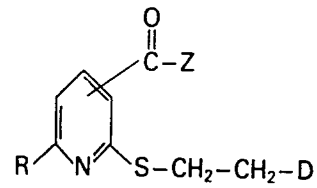

(IA)

dans laquelle

R représente un atome d'hydrogène ou un groupe alkyle en C1–C4,

Z représente un groupe phényle éventuellement substitué par un ou plusieurs atomes d'halogènes et/ou groupes alkyle en C1–C4,

D représente un groupe hydroxy ou un atome d'halogène, ou un groupe –NH–E dans lequel E représente un groupe répondant aux formules suivantes:

(a) 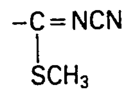

(b) 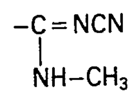 ou

(c) 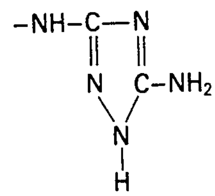

et le substituant

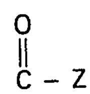

est relié en position 3 ou 4 du cycle de pyridine, et leurs sels formés par addition avec des acides.

2. Procédé de préparation des dérivés de la pyridine répondant à la formule générale IA

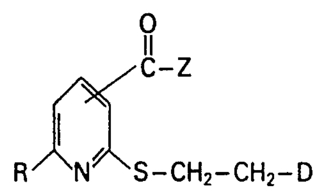

(IA)

dans laquelle

R représente un atome d'hydrogène ou un groupe alkyle en C1–C4,

Z représente un groupe phényle éventuellement substitué par un ou plusieurs atomes d'halogènes et/ou groupes alkyle en C1–C4,

D représente un groupe hydroxy ou un atome d'halogène, ou un groupe –NH–E dans lequel E représente un groupe répondant aux formules suivantes:

(a) 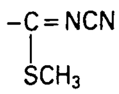

(b) 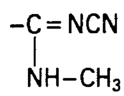 ou

(c) 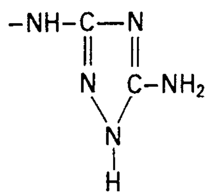

et le substituant

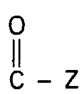

est relié en position 3 ou 4 du cycle de pyridine, et de leurs sels formés par addition avec des acides, caractérisé en ce que: a1) on fait réagir un composé de formule II

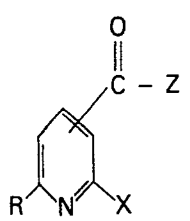

(II)

dans laquelle R et Z ont les significations indiquées ci-dessus et X représente un atome d'halogène, avec un thiol de formule générale III

$$HS-CH_2-CH_2-D \qquad (III)$$

dans laquelle D a les significations indiquées ci-dessus, ou avec un sel d'un tel composé formé par addition avec un acide; ou bien a2) on fait réagir une pyridine-2-thione de formule générale IV

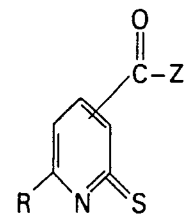

(IV)

dans laquelle Z et R ont les significations indiquées ci-dessus, avec un dérivé de 2-halogénoéthane de formule générale V

$$X - CH_2 - CH_2 -D \qquad (V)$$

dans laquelle D a les significations indiquées ci-dessus et X représente un atome d'halogène, ou avec un sel de ce composé formé par addition avec un acide, et si on le désire, on convertit un composé obtenu répondant à la formule générale IA en un sel formé par addition avec un acide ou on libère un tel composé à partir de l'un de ses sels d'addition avec un acide.

3. Procédé selon les revendications 1 et 2, variante a1), caractérisé en ce que la réaction est effectuée dans un solvant en présence d'un accepteur d'acide.

4. Procédé selon les revendications 1 et 2, variante a1), caractérisé en ce que la réaction est effectuée dans une solution concentrée d'acide chlorhydrique à une température correspondant au point d'ébullition du mélange.

5. Procédé selon les revendications 1 et 2, variante a2), caractérisé en ce que la réaction est effectuée dans un alcool inférieur en présence d'un hydroxyde de métal alcalin.

6. Procédé selon les revendications 1 et 2, pour la préparation des composés de formule générale IA dans laquelle D représente un groupe de formule générale –NH–E et E représente un groupe de formule (b), R et Z ayant les significations indiquées ci-dessus, caractérisé en ce que l'on fait réagir un composé de formule générale IA dans laquelle D représente un groupe –NH–E dans lequel E représente un groupe de formule (a), avec la méthylamine.

7. Procédé selon les revendications 1 et 2, pour la préparation des composés de formule générale IA dans laquelle D représente un groupe de formule générale –NH–E et E représente un groupe de formule (c), R et Z ayant les significations indiquées ci-dessus, caractérisé en ce que l'on fait réagir un composé de formule générale IA dans laquelle D représente un groupe –NH–E et E représente un groupe de formule (a), avec l'hydrate d'hydrazine.